Europäisches Patentamt

European Patent Office

Office européen des brevets

(11) Publication number: **0 027 651**
**B1**

# EUROPEAN PATENT SPECIFICATION

(45) Date of publication of patent specification: **10.04.85**

(21) Application number: **80106342.1**

(22) Date of filing: **17.10.80**

(51) Int. Cl.⁴: **G 02 B 23/00,** G 02 B 7/04, A 61 B 1/04

(54) **Endoscope ocular section.**

(30) Priority: **18.10.79 JP 134384/79**
**12.12.79 JP 161234/79**

(43) Date of publication of application:
**29.04.81 Bulletin 81/17**

(45) Publication of the grant of the patent:
**10.04.85 Bulletin 85/15**

(84) Designated Contracting States:
**AT BE DE FR GB IT NL SE**

(56) References cited:
**DE-A-3 008 502**
**JP-A-45 027 677**
**US-A-4 114 984**

(73) Proprietor: **OLYMPUS OPTICAL CO., LTD.**
**43-2, 2-chome, Hatagaya Shibuya-ku**
**Tokyo 151 (JP)**

(72) Inventor: **Yamaguchi, Tatuya**
**3-6-36, Sakae-cho Hino-shi**
**Tokyo (JP)**

(74) Representative: **Kinzebach, Werner, Dr. et al**
**Patentanwälte Reitstötter, Kinzebach und**
**Partner Postfach 780**
**D-8000 München 43 (DE)**

Courier Press, Leamington Spa, England.

## Description

This invention relates to improvements on an endoscope ocular section provided with a diopter-adjusting mechanism.

An endoscope ocular section having an ocular optical system applied to the examination and medical treatment of the coeliac cavity of a human body (hereinafter simply referred to as "the coeliac cavity") is generally provided with a diopter-adjusting mechanism for controlling the position of an ocular in accordance with the diopter of an observer's eye. Where the coeliac cavity is photographed by a photographic camera fitted to an endoscope, the ocular acts as a photographic lens itself or part thereof. Therefore, the ocular which has previously been shifted to match the diopter of the observer's eye has to be adjusted to a prescribed position to photograph the coeliac cavity. If this positioning operation is neglected, the resultant photograph of the coeliac cavity will be out of focus.

To avoid such difficulty, therefore it is desirable that the ocular can be automatically set in a position adapted to photograph the coeliac cavity simply by fitting a photographic camera to the ocular section of an endoscope. In this case, it is necessary to fit the photographic camera to the ocular section of the endoscope unfailingly and with a high precision.

An endoscope ocular section proposed in JP—A50—89333 in order to meet the above-mentioned requirements is the type wherein a cylindrical cam comprises a cam surface for allowing the lens mount of a photographic camera to be moved in accordance with the extent to which the diopter of the ocular has to be adjusted, and flat planes are formed on both sides of the cam surface, said flat planes being intended to set the ocular in such a position as to convert light beams emitted through an objective into converging or parallel rays; and the cylindrical cam is rotated to adjust the diopter of the objective and also produce converging or parallel rays as need arises. Where the cylindrical cam of the proposed endoscope ocular section is rotated, light beams conducted through the objective are carried beyond the cam surface of the cylindrical cam up to the flat planes on both sides thereof. In such case, the lens frame is undesirably shifted beyond the range in which the lens diopter is to be adjusted. If, under this condition, it is tried to fit the photographic camera to the ocular section, the cylindrical cam is not brought back to a position denoting a zero diopter, eventually resulting in the failure of the precise mounting of the photographic camera on the ocular section.

Another known endoscope (JP—A50—133849) ocular is the type wherein a slidable cylinder is moved along an optical axis by the rotation of a cylindrical cam fitted into a support cylinder; the slidable cylinder is fitted with a movable cylinder for shifting the ocular in the direction of the optical axis; the slidable cylinder is pressed against the cam surface of the cylindrical cam by a corresponding spring; the movable cylinder is pressed against the slidable cylinder similarly by a corresponding spring; and the movable cylinder is provided with an engagement section. Where, with the second proposed endoscope ocular section, it is tried to connect the connector of the photographic camera to the engagement section, the connector contacts the engagement section in different positions, according as the ocular indicates plus and minus diopters. Therefore, the ocular is slightly displaced from a prescribed position, probably causing a badly focused image to be impressed on a photographic film.

From JP—A—45 27 677 an endoscope ocular is known which comprises a cylindrical diopter adjusting cam which is fixedly connected to a diopter adjusting knob. The adjusting cam acts on an occular guiding pin which is connected to the ocular lens frame and which is urged by a spring against said cam. A cylindrical interlocking cam which is provided with an axially protruding pin is fitted to the ocular section coaxially with respect to said diopter adjusting cam. Both of said cams can be rotated with respect to the ocular section but are immovable in axial direction. When a photographic camera is fitted to the ocular section of the endoscope then the pin is rotated and therefore the interlocking cam rotates. During this rotation the inclined cam surface of said interlocking cam engages the ocular guiding pin so that the ocular is moved against the urging force of the spring. Simultaneously, the ocular guiding pin is lifted from the surface of the diopter adjusting cam. In this type of endoscope ocular section the ocular can be moved by the interlocking cam only to a position which corresponds to at least the maximum position of the diopter adjusting cam. It is not possible to bring the ocular to an intermediate position (i.e. the zero diopter position) if the diopter adjusting cam is set to the above-mentioned maximum position. This means that the camera to be connected to the endoscope must be designed so that the light is focused to the film in the above maximum diopter position.

The object of this invention is to provide an endoscope ocular section the diopter position of which is adjusted to any desired value within the adjusting range of the ocular adjusting mechanism independently of the position of the diopter adjusting mechanism.

To attain the above-mentioned object this invention provides an endoscope ocular section comprising an ocular support which is fixed to a control section of an endoscope and through which an image guide extends, a hollow cylindrical mount body which has two ends, one end being connected to said ocular support and the other end being provided with a photographic camera and which has a central bore, a lens mounting slidably fitted in said central bore of said mount body so as to approach and recede from said image guide, and an ocular held in said lens mounting, an axial slit formed in said mount body; an ocular guiding pin extending radially outward from said lens frame through said axial slit; an occular diopter adjusting mechanism with adjusting knob provided on said ocular support for moving said ocular axially with respect to said mount body, between a maximum and a minimum diopter position by means of said ocular guiding pin; and a preset diopter defining element which is surrounded by said mount body so as to be rotatable but axially immovable and which has at least one inclined cam surface for moving said ocular guiding pin to dispose said ocular in the preset diopter position and for permitting said preset diopter defining element to be brought to a position free from said ocular guiding pin, which is characterized in that the diopter adjusting mechanism comprises one intermediate member operatively connected between said adjusting knob and said ocular guiding pin, and being axially movable with respect to said mount body, and that said preset diopter defining element disconnects said connection of said intermediate member between said adjusting knob and said ocular guiding pin and simultaneously defines the preset position of said lens mounting irrespective of said ocular diopter adjusting mechanism, wherein said preset diopter position is between said maximum and said minimum diopter positions.

With the endoscope ocular embodying this invention, a mechanism for resetting the ocular in a zero diopter position is provided in addition to a diopter-adjusting mechanism, thus offering the advantage that the ocular can be reset to the zero diopter position unfailingly and easily by fitting the photographic camera to a prescribed position on the mount body, no matter what position the ocular may take, before it is reset to the zero diopter position.

This invention can be more fully understood from the following detailed description with reference to the accompanying drawings in which:

Fig. 1 is a lateral view of the whole of an endoscope provided with an ocular section according to a first embodiment of this invention;

Fig. 2 indicates an oblique view of the endoscope ocular section of Fig. 1 and that of the mount of a photographic camera coupled to the endoscope ocular section;

Fig. 3 is a longitudinal cross sectional view of Fig. 2;

Fig. 4 is an oblique view of a first cylindrical cam used in Fig. 3;

Fig. 5 is an oblique view of a second cylindrical cam used in Fig. 3;

Figs. 6 and 7 illustrate the operation of the first and second cylindrical cams of Fig. 3;

Fig. 8 is a lateral view of the whole of an endoscope provided with an ocular according to a second embodiment of the invention;

Fig. 9 shows an oblique view of the endoscope ocular used in Fig. 8 and that of a photographic camera fitted to the endoscope ocular section;

Fig. 10 is a longitudinal cross sectional view of Fig. 9;

Fig. 11 is an exploded oblique view of the diopter adjusting shaft assembly of Fig. 10 and its associated members;

Fig. 12 is an exploded oblique view of the actuating ring of Fig. 10 and cylindrical cam engageable therewith;

Fig. 13 shows the operation of the cylindrical cam of Fig. 10; and

Fig. 14 is a fractional sectional view of an endoscope ocular section according to a third embodiment of the invention.

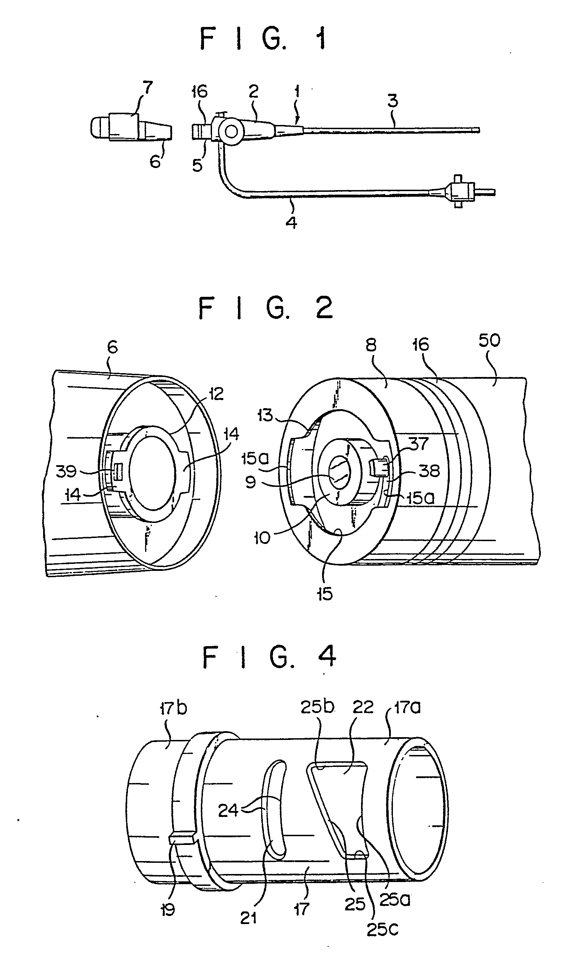

Referring to Fig. 1, an endoscope 1 comprises a control section 2, an insertion section 3 and a flexible umbilical cord 4 extending from the control section 2. The distal end of the insertion section 3 is provided with an observation window and illumination window (not shown). The observation window is optically connected to an ocular section 5 on the control section 2 through an image-transmitting optical system extending through the control section 2 and insertion section 3. The illumination window sends forth illumination light beams emitted from an external light source through an illumination light-transmitting optical system extending through the flexible umbilical cord 4. As described below, a photographic camera 7 can be fixed to the ocular section 5 of the control section 2 by means of a lens mount 6 of the camera 7.

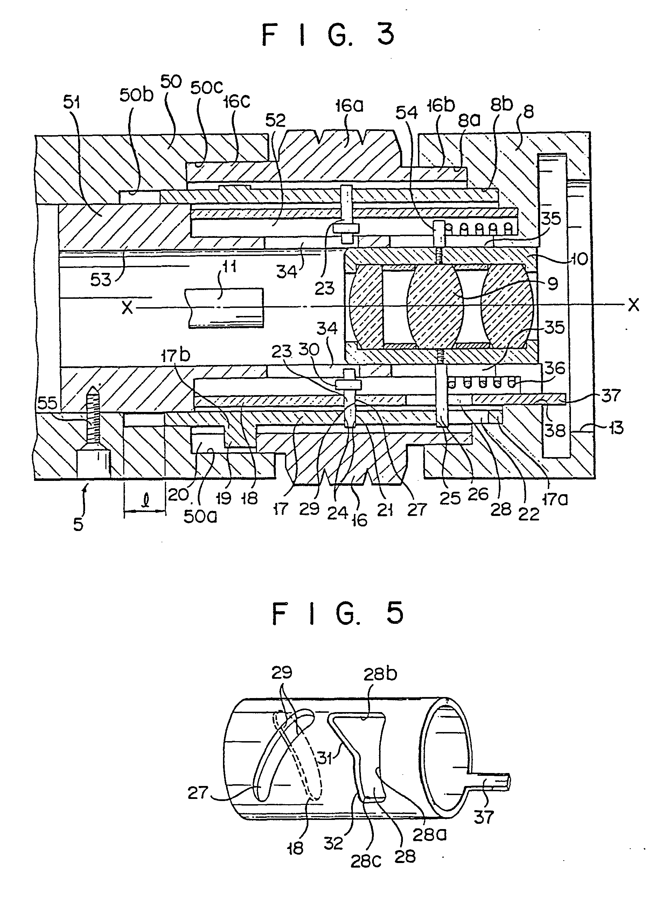

Referring to Figs. 2 and 3, the ocular section 5 is set in a hollow cylindrical mount body 8 in such a manner that a lens mount 10 holding an ocular 9 can be freely moved in the direction of the optical axis of the ocular section 5. The ocular 9 faces the end face of an image transmitting optical fiber bundle 11 constituting an image guide.

The distal end of the mount body 8 is provided with a receptacle 13 into which the bayonet 12 of the mount 6 of the photographic camera 7 is inserted. The receptacle 13 has an inner edge 15, in which a pair of notches 15a extending radially outward are formed to receive the pawls 14 of the bayonet 12. The photographic camera 7 is fitted to the mount body 8 through the steps of first fitting the pawls 14 of the bayonet 12 into the notches 15a and then rotating the bayonet 12 in a prescribed direction to effect engagement between the bayonet 12 and the inner edge 15.

A diopter adjusting ring 16 comprises a control knob 16a and hollow cylindrical sections 16b, 16c formed at the front and rear ends of the central knob 16a. The hollow cylindrical sections 16b, 16c are respectively engaged with a large diameter hole 8a formed in the mount body 8 and a large diameter hole 50a formed at the front end of an ocular support 50. The diopter adjusting ring 16 is rotatable about the optical axis X—X of the ocular 9 but immovable axially of the ocular 9.

A first cylindrical cam 17 extends through the diopter-adjusting ring 16. A second cylindrical cam 18 is concentrically fitted into the first cylindrical cam 17. Both first and second cylindrical cams 17, 18 should preferably be set concentric with the optical axis X—X.

The front end portion 17a of the first cylindrical cam 17 is fitted into a small diameter hole 8b of the mount body 8. The rear end portion 17b of the first cylindrical cam 17 is fitted into a small diameter hole 50b of the ocular support 50. The first cylindrical cam 17 is rotatable and also movable for a sufficient distance l to cover the whole range in which the diopter is to be axially adjusted, that is, in parallel with the optical axis X—X. An axially extending guide notch 20 is formed in the lateral wall of the rear hollow cylindrical section 16c of the diopter adjusting ring 16. An engagement section 19 projectively provided on the lateral wall of the rear end portion 17b of the first cylindrical cam 17 is fitted into the guide notch 20 (Fig. 3). The first cylindrical cam 17 is rotatable with the diopter adjusting ring 16 and further movable in the axial direction independently of the diopter-adjusting ring 16.

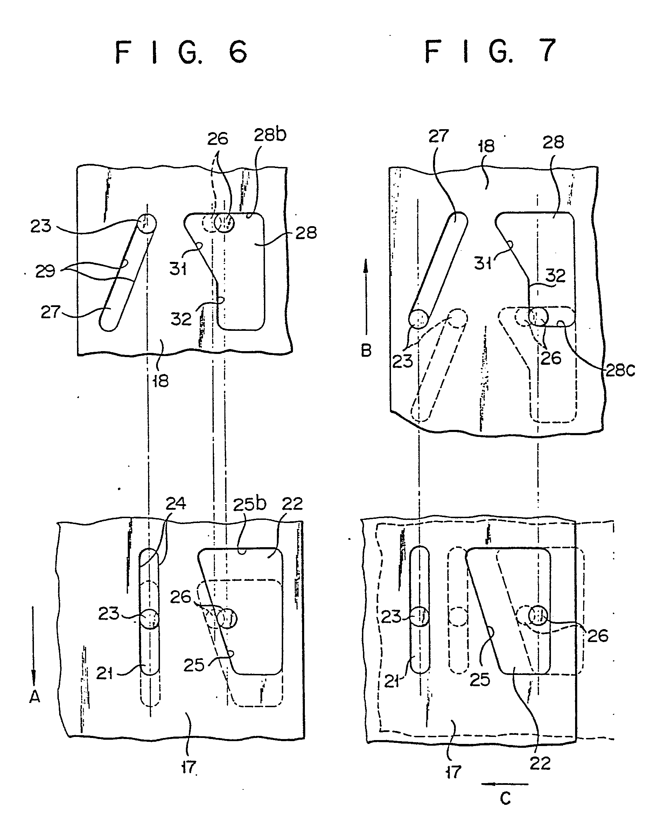

Referring to Fig. 4, there are provided on the lateral wall of the first cylindrical cam 17 a pair of guide slits 21 extending in the circumferential direction and diametrically facing each other and a trapezoidal opening 22 positioned near the front end portion of the first cylindrical cam 17 as viewed from the guide slits 21. The circumferentially extending guide slit 21 has substantially the same width as the outer diameter of the later described guide pin 23 inserted into the guide slit 21. Both edges of each guide slit 21 respectively act as cam surfaces 24. The trapezoidal opening 22 has a guide cam surface 25 which is near to the guide slit 21 and inclined to the axis of the first cylindrical cam 17 and which guides the later described ocular guiding pin 26. The edge 25a of the trapezoidal opening 22 opposite to the inclined guide cam surface 25 extends circumferentially of the first cylindrical cam 17. The other two edges 25b, 25c of the trapezoidal opening 22 extend axially of the first cylindrical cam 17.

Referring to Fig. 5, there are provided on the lateral wall of the second cylindrical cam 18 a pair of guide slits 27 which are inclined to the axis of the second cylindrical cam 18 in the opposite direction to that in which the guide cam surface 25 of the trapezoidal opening 22 of the first cylindrical cam 17 is inclined, and diametrically face each other; and an opening 28 positioned near the front edge of the second cylindrical cam 18 as viewed from the guide slits 27. The guide slit 27 has substantially the same width as the diameter of the guide pin 23 inserted into the guide slit 27 such that both edges of the guide slit 27 respectively act as a cam surface 29. The opening 28 comprises a cam surface 31 inclined toward the inclined cam surface 25 of the first cylindrical cam 17; a cam surface 32 which is bent from the cam surface 31 and extends circumferentially of the second cylindrical 18; a circumferentially extending edge 28a facing both cam surfaces 31, 32; and the other two axially extending edges 28b, 28c.

The mount body 8 has rearwardly extending small diameter sleeve 51. An annular cavity 52 is formed in the outer peripheral wall of the sleeve 51. The second cylindrical cam 18 is disposed in the annular cavity 52 with no room left at the front and back ends of said cavity 52. The lens mount 10 is reciprocatively inserted into a central cylindrical bore 53 of the mount body 8.

A pair of diametrically facing guide pins 25, 54 project outward from the outer peripheral wall of the lens frame 10 through a pair of axially extending slots 35 formed at the front end portion of the sleeve 51. Where the photographic camera 7 is taken off, the ocular section 5, the pin 25 protrudes into the opening 22 of the first cylindrical cam 17 without touching the cam surface 31 and other edges of the opening 28 of the second cylindrical cam 18. The pin 54 protrudes into the annular cavity 52 through the axially extending slot 35. A compression coil spring 36 is provided in the front end portion of the annular cavity 52 so as to surround the sleeve 51. This compression coil spring 36 normally rearwardly urges the lens frame 10 and ocular 9, one end of the spring 36 being pressed against the front wall of the annular cavity 52 and the other end thereof being pressed against the pins 25, 26. Where the photographic camera 7 is taken off the ocular section 5, the rear side of the peripheral wall of the head of the pin 26 touches the cam surface 25 of the first cylindrical cam 17, thereby preventing the lens frame 10 (that is, the ocular 9) from being carried rearward.

Another pair of pins 23 respectively pass through the two slits 21 of the first cylindrical cam 17 and the two slits 27 of the second cylindrical cam 18. The inner ends of the pins 23 protrude into slots 34 formed in the sleeve 51 of the mount body 8. Between the second cylindrical cam 18 and sleeve 51 the pins 23 are connected by a ring member 30 such as a nut which has a broader width than the pin 23 and slot 34, thereby being prevented from falling off the first and second cylindrical cams 17, 18. The rear end portion of the sleeve 51 of the mount body 8 is fixed into the front end portion of the ocular support 50 by means of a screw 55.

As shown in Figs. 3 and 5, a projection 37 for the rotation of the second cylindrical cam 18 axially projects from the front end of the second cylindrical cam 18. The projection 37 passes through arcuate slit 38 formed in the front wall of the mount body 8 and protrudes outward therefrom. Where the photographic camera 7 is fitted to the mount body 8, the projection 37 is inserted into an engagement hole 39 formed in the pawl 14 of the bayonet 12 (Fig. 2). Fig. 3 shows the condition of an endoscope ocular in which the ocular 9 proceeds most forward.

In operation (reference is made to Fig. 6), let it be assumed that where the ocular 9 is in a position denoting a zero diopter, the pins 23 contact the central portions of the slit 21 of the first cylindrical cam 17 and that end of the slit 27 of the second cylindrical cam 18 which is at the side of the edge 28b of the opening 28 of the second cylindrical cam 18, and the pin 26 contacts the central portion of the cam surface 25 of the first cylindrical cam 17 and the edge 28b of the opening 28 of the second cylindrical cam 18. Where, under this condition, the diopter-adjusting ring 16 (Fig. 3) is actuated to rotate the first cylindrical cam 17 in the direction of an arrow A (as shown in Fig. 6), the pin 26 in contact with the cam surface 25 is brought to a position indicated in a dotted circle by the force of the compression coil spring 36 without being obstructed by the cam surfaces 31, 32. As a result, the ocular 9 is drawn near to the image transmitting optical fiber bundle 11 by the extent to which the pin 26 is shifted. Similarly, where the diopter-adjusting ring 16 is rotated in the opposite direction to the above-mentioned case, the ocular 9 is removed from the image-transmitting optical bundle 11. Therefore, the position of the ocular 9 can be easily adjusted in conformity with the diopter of the observer's eye. In either case, the second cylindrical cam 18 is neither rotated nor axially moved.

Description is now given of the fitting of the photographic camera 7 to the ocular section 5. Now let it be assumed that the photographic camera 7 is fitted to the ocular section 5 with the position of the ocular 9 adjusted to the diopter of the observer's eye, and that the pin 26 occupied a position shown in a dotted circle in Fig. 6 before the fitting of the photographic camera 7.

The projection 37 of the mount body 8 is inserted into the engagement hole 39 of the bayonet 12 provided in the mount 6 of the photographic camera 7. The mount 6 is rotated in such a direction in which the pawl 14 engaged by the projection 37 causes the second cylindrical cam 18 to be moved in the direction of an arrow B (Fig. 7). The rotation of the second cylindrical cam 18 is continued, until the pin 23 is brought to the opposite end of the guide slit 27 to that with which the pin 23 is engaged as shown in Fig. 6. At the end of the above-mentioned rotation, the pawl 14 of the bayonet 12 is engaged with the opening edge 15 of the receptacle 13, and the lens mount 6 of the photographic camera 7 is coupled to the mount body 8.

Where the second cylindrical cam 18 is moved as described above, and its slit 27 and opening 28 are shifted from the dotted line position to the solid line position, the pin 23 is carried axially rearward of the second cylindrical cam 18 (toward the left side of Fig. 7). As a result, the first cylindrical cam 17 is retracted in the direction of an arrow C shown in Fig. 7 for the same distance as that which is covered by the pin 23 carried axially rearward.

The other pin 26 first contacts the inclined cam surface 31 of the opening 28 of the second cylindrical cam 18, and is carried forward against the urging force of the compression coil spring 36 by the rotation of the second cylindrical cam 18 and is brought to the position in which it contacts the cam surface 32. Finally, the pin 26 touches the edge 28c of the opening 28 without being moved in the axial direction, and occupies the solid line position of Fig. 6, causing the ocular 9 to be set in a position denoting the zero diopter. Where the ocular 9 has its position adjusted to a point remote from the zero diopter position as viewed from the image transmission optical fiber bundle 11, the pin 26 is pressed against the cam surface 31 of the opening 28 and then the circumferentially extending cam surface 32 of the opening 28 by the urging force of the compression coil spring 36. As a result, the ocular 9 is brought to the zero diopter position. In either case, the first cylindrical cam 17 is moved in the direction of the arrow C of Fig. 7. Accordingly, the pin 26 is separated from the cam surface 25.

As apparent from the foregoing description, the ocular 9 is brought exactly to the zero diopter position after the fitting of the photographic camera 7 to the ocular section 5, no matter what position the ocular 9 may occupy before the fitting of the photographic camera 7. Therefore, a photographed image is prevented from being out of focus. This means that it is unnecessary to adjust the ocular 9 to the zero diopter position before the fitting of the photographic camera 7 to the ocular section 5, offering the advantage of facilitating the operation of the ocular section 5.

If necessary, the ocular section 5 can be designed to set the ocular 9 to any other diopter than zero diopter in accordance with the photographic camera to be mounted.

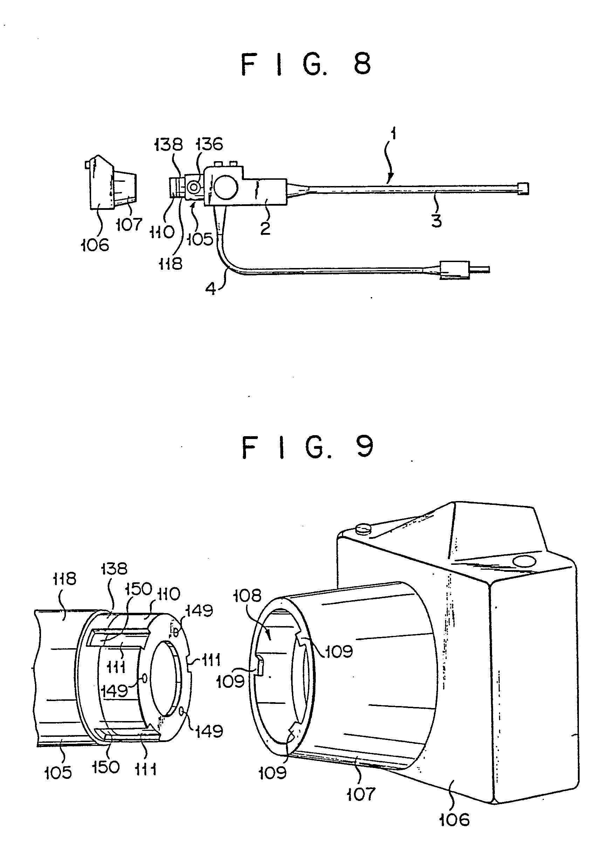

Description is now given with reference to Fig. 8 of an endoscope 1 provided with an ocular section 105 according to a second embodiment of this invention. With the second embodiment, the control section 2, insertion section 3 and flexible umbilical cord 4 of the endoscope 1 have the same construction and arrangement as those of the endoscope 1 of Fig. 1, description thereof being omitted. The ocular section 105 connected to the proximal end of the control section 2 is so constructed that a photographic camera 106 can be detachably fitted to the ocular section 105 as later described.

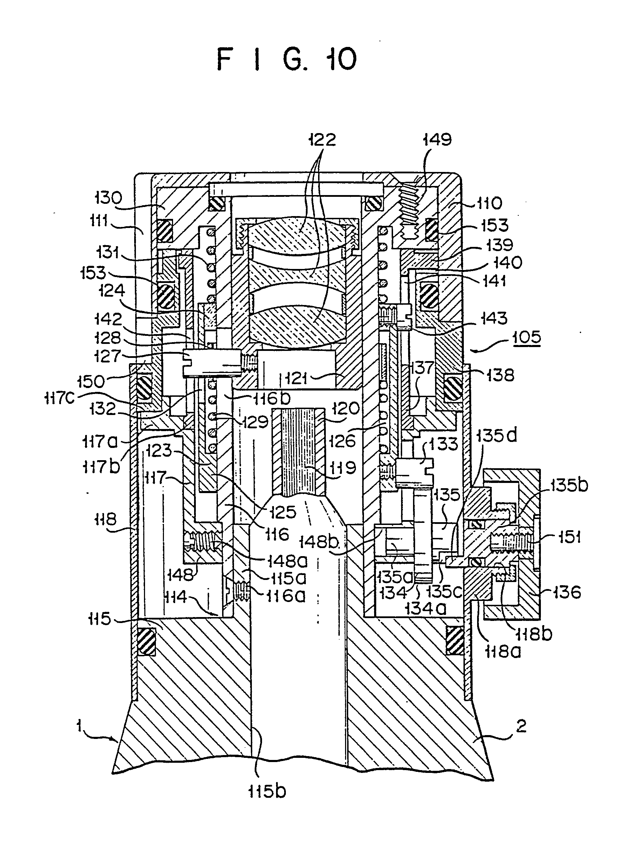

Referring to Figs. 9 and 10, the distal end portion of a lens mount 107 of the photographic camera 106 is provided with an annular bayonet receptacle 108 having pawls 109 equidistantly arranged in a circumferential direction.

The distal end portion of the ocular section 105 has a mount body 110 engageable with the bayonet receptacle 108. A plurality of axially extending bayonet grooves 111 are formed in the outer peripheral wall of the mount body 110. When the bayonet receptacle 108 of the photographic camera 106 is fitted to the mount body 110, the pawls 109 of the bayonet receptacle 108 are inserted into the grooves 111.

Description is now given with reference to Fig. 10 of the arrangement of the ocular section 105. An ocular support 114 formed on the ocular section 105 comprises a cylindrical, image guide holding portion 115 for an annular portion 115a concentrically projecting outward from the front end of the image guide holding portion 115. The mount body 110 includes a hollow cylindrical holding member 116 whose rear end is mounted on the annular portion 115a at the front end of the image holding portion 115 projects outward concentrically with the annular portion 115a. The hollow cylindrical holding member 116 is securely fixed to the annular member 115a by a set screw 116a. A hollow cylindrical support member 117 surrounds the rear half portion of the holding member 116. Formed on the rear end portion of the support member 117 is an inwardly extending flange 148 which is fixed to the rear end portion of the holding member 116 by means of a set screw 148a. A cylindrical outer casing 118 surrounds the distal end portion of the image guide holding portion 115 and the whole length of the support member 117. An image guide 119 formed of an image transmitting optical fiber bundle enclosed in a protective tube 120 passes through the central bore 115b of the holding portion 115 and concentrically projects into the holding member 116 at the front end of the annular portion 115a.

A lens mount 121 holding an ocular 122 is inserted into the front end portion of the holding member 116 in an axially slidable state on the central hole.

A cylindrical slider 123 surrounds the holding member 116. The rear end portion of the slider 123 has a slide section 125 whose inner wall contacts the outer peripheral wall of the holding member 116. The outer portion of the slide section 125 has an inner diameter larger than the outer diameter of the holding member 116. An annular space 126 is defined between the holding member 116 and slider 123. A ring 124 having an inner diameter substantially the same as the outer diameter of the holding member 116 is fixed to the front end portion of the slider 123. The ring 124 and slide section 125 cooperate to cause the slider 123 to axially reciprocate on the surface of the holding member 116.

An ocular guiding pin 127 projects outward from the lateral wall of the rear end portion of the lens frame 121 through axially extending slits 116b, 132 respectively formed in the peripheral wall of the holding member 116 and slider 123. An anchor ring 128 surrounding the holding member 116 is fixed to the pin 127, which penetrates the anchor ring 128.

A compression coil spring 129 surrounding the holding member 116 is stretched through the annular space 126. One end of the compression coil spring 129 is pressed against the inner wall of the slide section 148, and the other end of the compression coil spring 129 is pressed against the wall of that side of the anchor ring 128 which faces the slide section 148. The compression coil spring 129 normally urges the lens frame 121 and ocular 122 in the axial direction thereof through the pin 127, thereby to effect a contact between the front end of the slit 132 of the slider 123 and the peripheral wall of the pin 127.

Provided at the front end of the holding member 116 is an outward extending flange 130 which is covered with the mount body 110 and fixes it by means of a screw 149. A compression coil spring 131 surrounding the holding member 116 is stretched through a space defined between the rear end wall of the flange 130 and the front end wall of the ring 124. This compression coil spring 131 normally urges the slider 123 axially rearward.

Referring to Fig. 10, a cylindrical cam 137 surrounds the slider 123. The forward end of the cylindrical cam 137 is pressed against the rear end wall of the flange 130 of the holding member 116. The rear end of the cylindrical cam 137 is held in an annular groove 117b formed in the inner peripheral wall of an outward protruding flange 117a provided at the front end of the support member 117. Formed in the peripheral wall of the cylindrical cam 137 are the later described openings 141, 142 which respectively allow for the passage of a control pin 143 and the guide pin 127. The pin 143 concurrently carries out the later-described guiding function and fixes the ring 124 to the support member 117.

Referring to Figs. 10 and 12, an actuating ring 138 surrounds the cylindrical cam 137. One end of the actuating ring 138 is pressed against the rear end wall of the flange 130 of the holding member 116, and the other end of the actuating ring 138 is held in an annular groove 117c formed in the outer peripheral wall of the flange 117a of the support member 117. Axially extending notches 140 are formed in the front end wall of the actuating ring 138 and arranged in circumferentially equally spaced relation. Projections 139 formed on the outer peripheral wall of the front end portion of the cylindrical cam 137 are engaged with the corresponding notches 140, enabling the cylindrical cam 137 to be rotated with the actuating ring 138. Formed in the outer peripheral wall of the front end portion of the actuating ring 138 are axially extending grooves 150 constituting part of the bayonet grooves 111 (Figs. 9, 10 and 12).

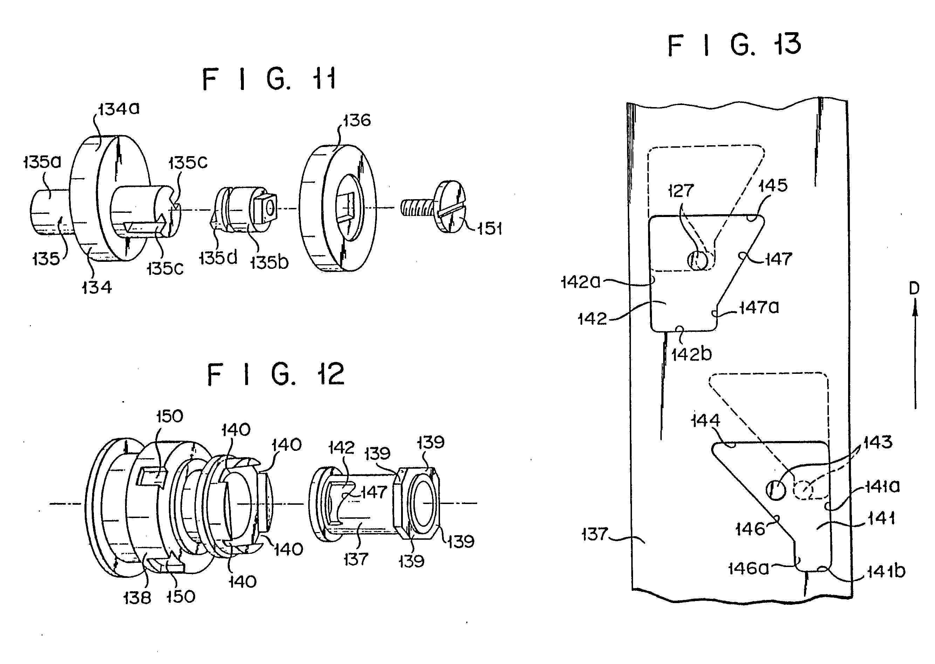

Referring to Fig. 10, a pin 133 projects radially outward from the rear end portion of the sliding member 125 of the slider 123. The peripheral wall of the pin 133 is contacted by a cam surface 134a of a diopter adjusting eccentric cam 134 which is rotated by a shaft assembly 135. Referring to Figs. 10 and 11, the shaft assembly 135 comprises a cam shaft 135a penetrating the eccentric cam 134 and a knob shaft 135b. Both shafts 135a, 135b are connected to each other when axially grooves 135c formed in the peripheral wall of one end portion of the cam shaft 135a receive projections 135d formed on one end of the knob shaft 135b. The other end of the cam shaft 135a is rotatably supported in a radially extending shaft hole 148b (Fig. 10) formed in the slide section 148 of the support member 117. A knob 136 is fixed to the other end of the knob shaft 135b by a set screw 151. The knob shaft 135b is rotatably fitted into a shaft hole 118b formed in a bearing section 118a provided at the rear end portion of the cylindrical outer casing 118.

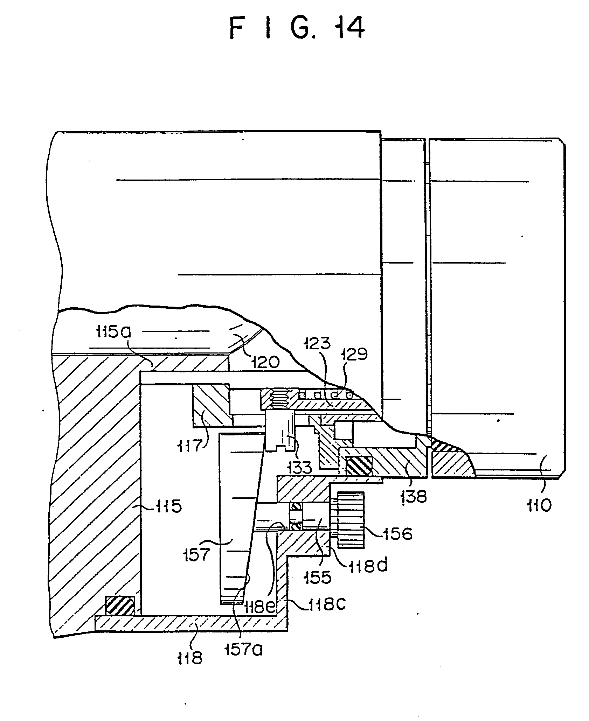

Referring to Fig. 13, the opening 141 formed in the cylindrical cam 137 is defined by a circumferentially extending edge 141a of the front end portion of the cylindrical cam 137; a bent edge consisting of an inclined cam surface 146 formed on the opposite side of the opening 141 to the circumferentially extending edge 141a and a circumferentially extending cam surface 146a formed contiguously to the inclined cam surface 146; a longer axially extending cam surface 144; and a shorter axially extending cam surface 141b. The opening 142 is defined by a circumferentially extending edge 142a of the rear end portion of the cylindrical cam 137; a bent edge formed of a cam surface 147 which is formed on the opposite side of the opening 142 to the circumferentially extending edge 142a and inclined in a direction opposite to that in which the cam surface 146 of the opening 141 of the cylindrical cam 137 is inclined and a circumferentially extending cam surface 147a formed contiguously the inclined cam surface 147; a longer axially extending cam surface 145; and a shorter axially extending cam surface 142b.

Where, in operation, the eccentric cam 134 is rotated to drive the pin 133 forward, the slider 123 advances to compress the compression coil springs 129 and 131. The compressed spring 129 causes the pin 127 to be carried forward while being pressed against the forward end of the slit 132. As a result, the ocular 122 is moved forward away from the image guide 119. Conversely where the eccentric cam 134 is rotated away from the pin 133, the compression coil spring 131 causes the slider 123 to be retracted, so that the pin 133 is pressed against the cam surface 134a of the eccentric cam 134. As a result, the pin 127 is pushed rearward by the slider 123, causing the ocular 122 to be drawn near to the image guide 119. Therefore, the ocular 122 can have its position adjusted in the axial direction to match the diopter of the observer's eye.

Description is now given of the process by which the ocular 122 is set in the zero diopter position.

Referring to Fig. 13, where the ocular 122 occupies the zero diopter position, the pin 127 occupies a dotted line position in which the peripheral wall of the pin 127 contacts a plane including the circumferentially extending cam surface 147a of the opening 142. Now let it be assumed that where the position of the ocular 122 is adjusted to match the diopter of the observer's eye, the pins 127, 143 are respectively set in a solid line position. Where, under this condition, the actuating ring 138 is rotated clockwise as viewed from the front of the mount body 110, the cylindrical cam 137 is moved in the direction of an arrow shown in Fig. 13. The pin 143 strikes against the inclined cam surface 146 of the opening 141, is gradually drawn near to the edge 141a, contacts the inclined cam surface 146a, and takes a dotted line position. The slider 123 advances against the urging force of the compression coil spring 131 to an extent corresponding to the above-mentioned movement of the pin 143. Though, at this time, the front end of the slit 132 tends to be removed from the pin 127, yet the pin 127 follows the movement of the slider 123 by the urging force of the compression coil spring 129. The pin 127 contacts the cam surface 147 of the opening 142, and then the circumferentially extending cam surface 147a when the cylindrical cam 137 is rotated in the direction of the arrow D, and finally occupies a dotted line position. As a result, the ocular 122 is again adjusted to the zero position by means of the pin 127 and lens frame 121.

Now let it be assumed that the ocular 122 is adjusted to a point remoter from the image guide 119 instead of the zero diopter position. Where the cylindrical cam 137 is rotated in the direction of the arrow D under the above-mentioned condition, then the pin 127 contacts the inclined cam surface 147 of the opening 142, and is brought to the circumferentially extending cam surface 147a by being guided along the inclined cam surface 147 to occupy the zero diopter position indicated in a dotted line circle. The cam surface 146 causes the slider 123 to advance against the urging force of the compression coil spring 131. Thus, the ocular 122 is adjusted to the zero diopter position. Where the photographic camera 106 is not mounted, axially extending grooves 150 formed in the peripheral wall of the front end portion of the actuating ring 138 is aligned with the bayonet grooves 11 of the mount body 110. Where, therefore, the pawls 109 of the camera mount 107 of the photographic camera 106 are engaged with the axial grooves 150 of the actuating ring 138, and the actuating ring 138 is rotated, the ocular 122 can be adjusted to occupy the zero diopter position without taking any particular means.

Description is now given with reference to Fig. 14 of a modification of the embodiment of Figs. 9 to 13. The modification of Fig. 14 only relates to the diopter adjusting cam mechanism of the embodiment. The other parts of Fig. 14 are the same as those of the embodiment of Figs. 9 to 13.

The modified diopter adjusting cam mechanism comprises: a cam shaft 155 rotatably supported in a shaft hole 118e formed in a bearing 118d provided in the front wall 118c of the cylindrical outer casing 118 in parallel with the slider 123; a knob 156 fixed to the outer end of the cam shaft 155; and a disc cam 157 whose center is fixed to the inner end of the cam shaft 155 and which has an inclined cam surface 157a whose peripheral portion touches the peripheral wall of a pin 133.

When the knob 156 is rotated, the slider 123 is axially moved against the urging force of the spring 131 in accordance with the shift due to the cam surface 157a, i.e., the head through which that portion of the inclined cam surface 157a which contacts the pin 133 is lifted. The above-mentioned axial movement of the slider 123 allows for the adjustment of the diopter of the ocular 122.

**Claims**

1. An endoscope ocular section comprising an ocular support (50, 114) which is fixed to a control section (2) of an endoscope (1) and through which an image guide (11, 119) extends, a hollow cylindrical mount body (8, 116) which has two ends, one end being connected to said ocular support (50, 114) and the other end being provided with a photographic camera (7, 106) and which has a central bore (53), a lens mounting (10, 121) slidably fitted in said central bore of said mount body (8, 116) so as to approach and recede from said image guide (11, 119) and an ocular (9, 122) held in said lens mounting (10, 121), an axial slit (35, 116b) formed in said mount body (8, 116); an ocular guiding pin (26, 127) extending radially outward from said lens mounting (10, 121) through said axial slit; an ocular diopter adjusting mechanism with adjusting knob (16, 136) provided on said ocular support (50, 114) for moving said ocular (9, 122) axially with respect to said mount body (8, 116) between a maximum and a minimum diopter position by means of said ocular guiding pin (26, 127); and a preset diopter defining element (18, 137) which is surrounded by said mount body (8, 116) so as to be rotatable but axially immovable and which has at least one inclined cam surface (31, 146, 147) for moving said ocular guiding pin (26, 127) to dispose said ocular (9, 122) in the preset diopter position and for permitting said preset diopter defining element (18, 137) to be brought to a position free from said ocular guiding pin (26, 127) characterized in that the diopter adjusting mechanism comprises one intermediate member (19, 125) operatively connected between said adjusting knob (16, 136) and said ocular guiding pin (26, 127) and being axially movable with respect to said mount body (8, 116), and that said preset diopter defining element (18, 137) disconnects said connection of said intermediate member (17, 125) between said adjusting knob and said ocular guiding pin and simultaneously defines the preset diopter position of said lens mounting (10, 121) irrespective of said ocular diopter adjusting mechanism, wherein said preset diopter position is between said maximum and said minimum diopter positions.

2. The endoscope ocular section according to claim 1, characterized in that said preset diopter defining element is a first rotatable cylindrical cam (18), provided with said inclined cam surface (31); and said intermediate member is a second rotatable cylindrical cam (17) which surrounds said first cylindrical cam (18), is axially movable, and has an inclined cam surface (25); said diopter adjusting knob (16) being ring-shaped, which surrounds said second rotatable cylindrical cam (17) and, when rotated, causes to rotate said second rotatable cylindrical cam (17), and spring means (36) provided in said mount body (8) for urging said ocular guiding pin (26) against said inclined surface (25) of said second rotatable cylindrical cam (17).

3. The endoscope ocular section according to claim 2, characterized in that said first rotatable cylindrical cam (18) has a first opening (28) allowing for passage of said ocular guiding pin (26); the second rotatable cylindrical cam (17) has a second opening (22) similarly allowing for passage of said ocular guiding pin (26) and said inclined cam surfaces (31, 25) are respectively formed on said first (28) and second openings (22).

4. The endoscope ocular section according to claim 3, characterized in that said first opening (28) further comprises a circumferentially extending cam surface (28a) which is contiguously connected to said inclined cam surface (31) of the first rotatable cylindrical cam (18) at the downstream side of its rotation, and, when contacted by the ocular guiding pin (26) adjusts said ocular (9) to the zero diopter position.

5. The endoscope ocular section according to claim 4, characterized in that said first rotatable cylindrical cam (18) is provided with an inclined cam (29) said second rotatable cylindrical cam (17) is provided with a circumferentially extending slit (21), a slit pin (23) passes through said slits (21, 27) of both cylindrical cams (17, 18) at the same time, and said inclined slit (27) is inclined in such a direction that said second rotatable cylindrical cam (17) is axially moved so as to separate said ocular guiding pin (26) from said inclined cam (25) surface of said second rotatable cylindrical cam (17) when said first rotatable cylindrical cam (18) is rotated.

6. The endoscope ocular section according to claim 5, characterized in that said diopter adjusting ring (16) has an axially extending slit (20) and said second rotatable cylindrical cam (17) has a projecting engagement section (19) which engages said slit (20) of said diopter adjusting ring (16) so that the second cam (17) can be moved with respect to ring (16) only in axial direction.

7. The endoscope ocular section according to claim 1, characterized in that an outer cylindrical housing (118) surrounding the mount body (116) is fixed to the ocular support (114); said intermediate member being formed as

a hollow cylindrical slider (125) which is provided with an axially extending slit (132) allowing for insertion of said ocular guiding pin (127) and which is axially movable with respect to the mount body (116);

a first compression coil spring (129) which is set in said slider (125) urging said ocular guiding pin (127) toward that end of said slit (132) of said slider (125) which faces said other end of said mount body (116);

a diopter adjusting pin (133) extending radially outward from said slider (125);

a diopter adjusting cam assembly comprising a cam shaft (135a, 155) which is operatively connected to said adjusting knob (136) being rotatably mounted on said outer cylindrical casing (118) and an adjusting cam (134, 157) which is fixed to said cam shaft (135a, 155) contacts said diopter adjusting pin (133) to move said slider (125) axially via said diopter adjusting pin (133) when said cam shaft (135a, 155) is rotated; and

a second compression spring (131) which is fitted to said mount body (116) and urges said slider (125)

against said adjusting cam (134, 157) via said

# 0 027 651

diopter adjusting pin (133).

8. The endoscope ocular section according to claim 7, characterized in that said cam shaft (135a) is fitted to said outer cylindrical casing (118) to extend radially out of said mount body (116); and the cam (134) is a disc-shaped eccentric cam.

9. The endoscope ocular section according to claim 7, characterized in that said cam shaft (155) extends axially out of said mount body (116); and said adjusting cam (157) has one end plane (157a) which is inclined with respect to said cam shaft (155) and is contacted by said diopter-adjusting pin (133).

10. The endoscope ocular section according to claim 9, characterized in that said preset diopter defining element is a cylindrical cam (137) surrounding said slider (125), and is provided with a first opening (142) whose edge facing said one end of the mount body (116) constitutes a first inclined cam surface (147), a second opening (141) whose edge facing the other end of the mount body (116) constitutes a second inclined cam surface (146) inclined in the opposite direction to said first inclined cam surface (147) and a control pin (143) which passes through the second opening (141) and is fixed to said slider (125); and both inclined cam surfaces (146, 147) are arranged so that said ocular guiding pin (127) is set in a position causing said ocular (122) to occupy a zero diopter position at the rotation downstream end.

## Revendications

1. Section oculaire d'endoscope comprenant un support (50, 114) d'oculaire qui est fixé à une section de commande (2) d'un endoscope (1) et à travers lequel s'étend un guide d'image (11, 119), un corps de montage cylindrique creux (8, 116) qui présente deux extrémités, une extrémité étant assemblée audit support (50, 114) d'oculaire et l'autre extrémité étant pourvue d'une chambre photographique (7, 106) et qui présente un alésage central (53), un porte-lentilles (10, 121) adapté à coulissement dans ledit alésage central dudit corps de montage (8, 116) de façon à s'approcher et s'éloigner dudit guide d'image (11, 119) et un oculaire (9, 122) retenu dans ledit porte-lentilles (10, 121), une fente axiale (35, 116b) formée dans ledit corps de montage (8, 116); une cheville de guidage (26, 127) d'oculaire s'étendant radialement vers l'extérieur à partir dudit porte-lentilles (10, 121) à travers ladite fente axiale; un mécanisme de réglage dioptrique de l'oculaire comportant un bouton de réglage (16, 136) prévu sur ledit support (50, 114) d'oculaire pour déplacer ledit oculaire (9, 122) axialement par rapport audit corps de montage (8, 116) entre une position dioptrique maximale et une position dioptrique minimale au moyen de ladite cheville de guidage (26, 127) d'oculaire; et un élément (18, 137) définissant un dioptre prédéterminé qui est entouré par ledit corps de montage (8, 116) de façon à pouvoir tourner sans être déplacé axialement et qui comporte au moins une surface de came inclinée (31, 146, 147) pour déplacer ladite cheville de guidage (26, 127) d'oculaire en vue de disposer ledit oculaire (9, 122) dans la position dioptrique prédéterminée et pour permettre audit élément (18, 137) définissant un dioptre prédéterminé d'être amené en une position libre par rapport à ladite cheville de guidage (26, 127) d'oculaire, caractérisée par le fait que le mécanisme de réglage dioptrique comprend un organe intermédiaire (17, 125) relié de manière active audit bouton de réglage (16, 136) et à ladite cheville de guidage (26, 127) d'oculaire et étant axialement déplaçable par rapport audit corps de montage (8, 116), et que ledit élément (18, 137) définissant un dioptre prédéterminé interrompt ladite liaison entre ledit organe intermédiaire (17, 125) et ledit bouton de réglage et ladite cheville de guidage d'oculaire, et définit simultanément la position dioptrique prédéterminée dudit porte-lentilles (10, 121) indépendamment dudit mécanisme de réglage dioptrique de l'oculaire, ladite position dioptrique prédéterminée étant située entre lesdites positions dioptriques maximale et minimale.

2. Section oculaire d'endoscope selon la revendication 1, caractérisée par le fait que ledit élément définissant un dioptre prédétetminé est une première came cylindrique rotative (18), pourvue de ladite surface de came inclinée (31); et que ledit organe intermédiaire est une seconde came cylindrique rotative (17) qui entoure ladite première came cylindrique (18), est axialement déplaçable et a une surface de came inclinée (25); ledit bouton de réglage dioptrique (16) étant de forme annulaire, qui entoure ladite seconde came cylindrique rotative (17) et, lorsqu'on le tourne, fait tourner ladite seconde came cylindrique rotative (17), un moyen à ressort (36) étant prévu dans ledit corps de montage (8) pour solliciter ladite cheville de guidage (26) d'oculaire contre ladite surface inclinée (25) de ladite seconde came cylindrique rotative (17).

3. Section oculaire d'endoscope selon la revendication 2, caractérisée par le fait que ladite première came cylindrique rotative (18) présente une première ouverture (28) autorisant le passage de ladite cheville de guidage (26) d'oculaire; la seconde came cylindrique rotative (17) présente une seconde ouverture (22) autorisant similairement le passage de ladite cheville de guidage (26) d'oculaire et lesdites surfaces de came inclinées (31, 25) sont respectivement formées sur lesdites première (28) et seconde (22) ouvertures.

4. Section oculaire d'endoscope selon la revendication 3, caractérisée par le fait que ladite première ouverture (28) comprend en outre une surface de came (28a) s'étendant circonférentiellement qui est reliée de façon contigue à ladite surface de came inclinée (31) de la première came cylindrique rotative (18) du côté aval de sa rotation, et, qui, lorsqu'elle vient en contact avec la cheville de guidage (26) d'oculaire règle ledit oculaire (9) en position dioptrique zéro.

5. Section oculaire d'endoscope selon la revendication 4, caractérisée par le fait que ladite

9

première came cylindrique rotative (18) est pourvue d'une came inclinée (29), ladite seconde came cylindrique rotative (17) est pourvue d'une fente (21) s'étendant circonférentiellement, une goupille de fente (23) passant à travers lesdites fentes (21, 27) des deux cames cylindriques (17, 18) en même temps, et ladite fente inclinée (27) est inclinée dans une direction telle que ladite seconde came cylindrique rotative (17) est axialement déplacée de façon à écarter ladite cheville de guidage (26) d'oculaire de ladite surface de came (25) inclinée, prévue sur ladite seconde came cylindrique rotative (17), lorsque ladite première came cylindrique rotative (18) vient à tourner.

6. Section oculaire d'endoscope selon la revendication 5, caractérisée par le fait que ledit premier anneau de réglage dioptrique (16) présente une fente (20) s'étendant axialement et que ladite seconde came cylindrique rotative (17) présente une section d'engagement (19) saillante qui vient s'engager dans ladite fente (20) dudit anneau de réglage dioptrique (16) de façon que la seconde came (17) puisse être déplacée seulement en direction axiale par rapport à l'anneau (16).

7. Section oculaire d'endoscope selon la revendication 1, caractérisée par le fait qu'un boîtier cylindrique extérieur (118) entourant le corps de montage (116) est fixé au support (114) d'oculaire; ledit organe intermédiare étant formé par

une douille coulissante cylindrique creuse (125) qui est pourvue d'une fente (132) s'étendant axialement et autorisant l'insertion de ladite cheville de guidage (127) d'oculaire et qui est axialement déplaçable par rapport au corps de montage (116);

un premier ressort hélicoïdal de compression (129) qui est logé dans ladite douille (125) de façon à pousser ladite cheville de guidage (127) d'oculaire vers l'extrémité de ladite fente (132) de ladite douille (125) qui est en regard de ladite autre extrémité dudit corps de montage (116);

un cheville de réglage dioptrique (133) s'étendant radialement vers l'extérieur à partir de ladite douille (125);

un ensemble à came de réglage dioptrique comprenant un arbre à came (135a, 155) qui est relié de façon active audit bouton de réglage (136), lequel est monté à rotation sur ledit boîtier cylindrique extérieur (118), et une came de réglage (134, 157) qui est fixée audit arbre à came (137a, 155), et entre en contact avec ladite cheville de réglage dioptrique (133) de façon à déplacer ladite douille (125) axialement par l'intermédiaire de ladite cheville de réglage dioptrique (133) lorsque ledit arbre à came (135a, 155) vient à tourner; et

un second ressort de compression (131) qui est adapté sur ledit corps de montage (116) et pousse ladite douille (125)

contre ladite came de réglage (134, 157) par l'intermédiaire de ladite cheville de réglage dioptrique (133).

8. Section oculaire d'endoscope selon la revendication 7, caractérisée par le fait que ledit arbre à came (135a) est adapté sur ledit boîtier cylindrique extérieur (118) de façon à s'étendre radialement en se prolongeant à l'extérieur dudit corps de montage (116); et que la came (134) est une came excentrique en forme de disque.

9. Section oculaire d'endoscope selon la revendication 7, caractérisée par le fait que ledit arbre à came (155) s'étend axialement à l'extérieur dudit corps de montage (116); et que ladite came de réglage (157) présente un plan terminal (157a) qui est incliné par rapport audit arbre à came (155) et vient en contact avec ladite cheville de réglage dioptrique (133).

10. Section oculaire d'endoscope selon la revendication 9, caractérisée par le fait que ledit élément définissant un dioptre prédéterminé est une came cylindrique (137) entourant ladite douille (125), et est pourvu d'une première ouverture (142) dont le bord en regard de ladite extrémité du corps de montage (116) constitue une première surface de came inclinée (147), d'une seconde ouverture (141) dont le bord en regard de l'autre extrémité du corps de montage (116) constitue une seconde surface de came inlinée (146), inclinée dans la direction opposée à ladite première surface de came inclinée (147), une cheville de réglage (143) passant à travers la seconde ouverture (141) et étant fixée à ladite douille (125); et que les deux surfaces de came inclinées (146, 147) sont disposées de façon que ladite cheville de guidage (127) d'oculaire soit réglée dans une position entraînant l'occupation par ledit oculaire (122) d'une position dioptrique zéro à la fin de la rotation en aval.

**Patentansprüche**

1. Endoskopokularteil einem Okularträger (50, 114), der am Steuerabschnitt (2) eines Endoskops (1) befestigt ist und durch den sich ein Bildleiter (11, 119) erstreckt, einem hohlen, zylindrischen Körper (8, 116), der zwei Enden aufweist, von denen das eine mit dem Okularträger (50, 114) verbunden ist und das andere mit einer photographischen Kamera (7, 106) versehen ist und eine zentrale Bohrung (53) aufweist, und wobei in die zentrale Bohrung des Körpers (8, 116) eine Objektivfassung (10, 121) gleitend eingepaßt ist, so daß sie sich dem Lichtleiter (11, 119) nähern und von ihm wieder entfernen kann, einem in der Objektivfassung (10, 121) gehaltenen Okular (9, 122), einem im Körper (8, 116) ausgebildeten axialen Schlitz (35, 116b), einem Okularführungszapfen (26, 127), der sich durch den axialen Schlitz von der Objektivfassung (10, 121) aus radial nach außen erstreckt, einem Okular-Dioptrieneinstellmechanismus mit einem Einstellknopf (16, 136) am Okularträger (50, 114) zur axialen Verschiebung des Okulars (9, 122) bezüglich des Körpers (8, 116) zwischen einer maximalen und minimalen Dioptrieneinstellung mit Hilfe des Okularführungszapfens (26, 127) und einem Dioptrienvoreinstellelement (18, 137), das vom Körper (8, 116) umschlossen ist, so daß es drehbar, aber axial unbeweglich ist und mindestens eine geneigte Steuerfläche (31, 146, 147) aufweist, die zum Verschieben des Okularführungszapfens (26, 127) dient, um das Okular (9, 122) in die vorgegebene Dioptrieneinstellung zu bringen und um zu erlauben daß das Dioptrienvoreinstellelement in eine Stellung gebracht werden kann, bei der es vom Okularführungszapfen (26, 127) gelöst ist, dadurch gekennzeichnet, daß der Dioptrieneinstellmechanismus ein Zwischenglied (17, 125) umfaßt, das in Wirkverbindung zwischen dem Einstellknopf (16, 136) und dem Okularführungszapfen (26, 127) angeordnet ist und bezüglich des Körpers (8, 116) axial beweglich ist, und daß das Dioptrienvoreinstellelement (18, 137) den Verbund des Zwischengliedes (17, 125) zwischen dem Einstellknopf und dem Okularführungszapfen unterbricht und gleichzeitig, unabhängig vom Okular-Dioptrieneinstellmechanismus, die vorgegebene Dioptrieneinstellung der Objektivfassung (10, 121) definiert, wobei die vorgegebene Dioptrieneinstellung zwischen der maximalen und minimalen Dioptrieneinstellung liegt.

2. Endoskopokularteil nach Anspruch 1, dadurch gekennzeichnet, daß das Dioptrienvoreinstellelement ein erstes, drehbares, zylindrisches Steuerelement (18) ist, das mit der geneigten Steuerfläche (31) versehen ist und daß das Zwischenglied ein zweites, drehbares, zylindrisches Steuerelement (17) ist, welches das erste zylindrische Steuerelement (18) umschließt, axial verschiebbar ist und eine geneigte Steuerfläche (25) aufweist, wobei der Dioptrien-Einstellknopf (16) ringförmig ist, das zweite, drehbare, zylindrische Steuerelement (17) umschließt und beim Drehen dazu führt, daß das zweite, drehbare, zylindrische Steuerelement (17) gedreht wird, und wobei im Körper (8) Federvorrichtungen (36) vorgesehen sind, um den Okularführungszapfen (26) gegen die geneigte Fläche (25) des zweiten, drehbaren, zylindrischen Steuerelements (17) zu drücken.

3. Endoskopokularteil nach Anspruch 2, dadurch gekennzeichnet, daß das erste, drehbare, zylindrische Steuerelement (18) eine erste Öffnung (28) aufweist, welche den Durchgang des Okularführungszapfens (26) erlaubt, daß das zweite, drehbare, zylindrische Steuerelement (17) eine zweite Öffnung (22) aufweist, die in gleicher Weise den Durchgang des Okularführungszapfens (26) erlaubt und daß die geneigten Steuerflächen (31, 25) jeweils an den ersten (28) bzw. zweiten (22) Öffnungen ausgebildet sind.

4. Endoskopokularteil nach Anspruch 3, dadurch gekennzeichnet, daß die erste Öffnung (28) zusätzlich eine umlaufende Steuerfläche (28a) aufweist, welche unmittelbar mit der geneigten Steuerfläche (31) des ersten, drehbaren, zylindrischen Steuerelements .(18) an der abwärts gerichteten Seite seiner Drehung verbunden ist und im Kontakt mit dem Okularführungszapfen (26) das Okular (9) in die O-Dioptrieneinstellung bringt.

5. Endoskopokularteil nach Anspruch 4, dadurch gekennzeichnet, daß das erste, drehbare, zylindrische Steuerelement (18) mit einer geneigten Steuerfläche (29) versehen ist und daß das zweite, drehbare zylindrische Steuerelement (17) mit einem sich in Umfangsrichtung erstreckenden Schlitz (21) versehen ist, wobei ein Schlitzzapfen (23) durch die Schlitze (21, 27) beider zylindrischer Steuerelemente (17, 18) gleichzeitig hindurchgeht und daß der geneigte Schlitz (27) in eine solche Richtung geneigt ist, daß das zweite, drehbare, zylindrische Steuerelement (17) axial so bewegt wird, daß der Okularführungszapfen (26) von der geneigten Steuerfläche (25) des zweiten, drehbaren zylindrischen Steuerelements (17) gelöst wird, wenn das erste, drehbare zylindrische Steuerelement (18) gedreht wird.

6. Endoskopokularteil nach Anspruch 5, dadurch gekennzeichnet, daß der Dioptrien-Einstellring (16) einen sich axial erstreckenden Schlitz (20) aufweist und daß das zweite, drehbare zylindrische Steuerelement (17) einen vorstehenden Eingriffsbereich (19) aufweist, welcher in den Schlitz (20) des Dioptrieneinstellrings (16) eingreift, so daß das zweite Steuerelement (17) bezüglich des Rings (16) nur in axialer Richtung bewegt werden kann.

7. Endoskopokularteil nach Anspruch 1, dadurch gekennzeichnet, daß ein äußeres, zylindrisches Gehäuse (118), welches den Körper (116) umschließt, mit dem Okularträger (114) verbunden ist, daß das Zwischenglied als hohler, zylindrischer Schieber (125) ausgebildet ist, der mit einem sich axial erstreckenden Schlitz (132) versehen ist, in welchen der Okularführungszapfen (127) eingesetzt werden kann und der bezüglich des Körpers (116) axial beweglich ist; daß eine erste Druckschraubenfeder (129), welche in den Schieber (125) eingesetzt ist und den Okularführungszapfen (127) nach dem Ende des Schlitzes (132) des Schiebers (125) drückt, welches dem anderen Ende des Körpers (116) gegenüberliegt, ein Dioptrieneinstellzapfen (133), der sich radial aus dem Schieber (125) nach außen erstreckt, ein Dioptrieneinstell-Steueranordnung mit einer Steuerwelle (135a, 155), welche in Wirkverbindung mit dem Einstellknopf (136) steht, der drehbar auf dem äußeren, zylindrischen Gehäuse (118) montiert ist und wobei eine Einstellnocke (134, 157), welche auf der Steuerwelle (135a, 155) befestigt ist, den Dioptrieneinstellzapfen (133) berührt, um den Schieber (125) axial zum Dioptrieneinstellzapfen (133) zu verschieben, sobald die Steuerwelle (135a, 155) gedreht wird und daß eine zweite Druckschraubenfeder (131) vorgesehen ist, welche auf den Körper (116) aufgesetzt ist und den Schieber (125) über den Dioptrieneinstellzapfen (133) gegen die Einstellnocke (134, 157) drückt.

8. Endoskopokularteil nach Anspruch 7, dadurch gekennzeichnet, daß die Steuerwelle (135a) am äußeren zylindrischen Gehäuse (118) so befestigt ist, daß sie radial vom Körper (116) fortragt und daß die Nocke (134) eine scheibenförmige, exzentrische Nocke ist.

9. Endoskopokularteil nach Anspruch 7, dadurch gekennzeichnet, daß die Steuerwelle (155) axial vom Körper (116) fortragt und daß die Einstellnocke (157) eine bezüglich der Steuerwelle (155) geneigte Endfläche (157) aufweist und vom Dioptrieneinstellzapfen (133) berührt wird.

10. Endoskopokularteil nach Anspruch 9, dadurch gekennzeichnet, daß das Dioptrienvoreinstellelement ein zylindrisches Steuerelement (137) ist, welches den Schieber (125) umschließt und mit einer ersten Öffnung (142) versehen ist, deren dem Körper (116) gegenüberliegender Rand eine erste, geneigte Steuerfläche (147) bildet, sowie einer zweiten Öffnung (141), deren dem anderen Ende des Körpers (116) gegenüberliegender Rand eine zweite, geneigte Steuerfläche (146) bildet, welche bezüglich der ersten geneigten Steuerfläche (147) in die umgekehrte Richtung geneigt ist, sowie einem Steuerzapfen (143), der sich durch die zweite Öffnung (141) erstreckt und am Schieber (125) befestigt ist, wobei beide geneigten Steuerflächen (146, 147) so angeordnet sind, daß der Okularführungszapfen (127) in eine Stellung gebracht wird, welche dazu führt, daß das Okular (122) am abwärts gerichteten Ende der Drehung eine O-Dioptrieneinstellung einnimmt.

F I G. 1

F I G. 2

F I G. 4

1

# F I G. 3

# F I G. 5

2

# F I G. 6

# F I G. 7

# F I G. 8

# F I G. 9

# F I G. 10

F I G. 11

F I G. 12

F I G. 13

# F I G. 14